# EUROPEAN PATENT SPECIFICATION

(11) **EP 4 389 029 B1**
(45) Date of publication and mention of the grant of the patent: **09.07.2025**
(21) Application number: 23150666.8
(22) Date of filing: 09.01.2023
(51) Int. Cl.: A61B 17/66, A61B 17/88, A61B 90/00

(54) **TOOL FOR EXTERNAL FIXATION STRUT ADJUSTMENT**
WERKZEUG ZUR EXTERNEN FIXATIONSSTREBENEINSTELLUNG
OUTIL POUR LE RÉGLAGE D'UNE ENTRETOISE DE FIXATION EXTERNE

(30) Priority: 23.12.2022 US 202263477067 P
(43) Date of publication of application: 26.06.2024
(60) Divisional application: 24217192.4
(73) Proprietor: Orthofix S.r.l., 37012 Bussolengo (VR) (IT); TEXAS SCOTTISH RITE HOSPITAL FOR CHILDREN, Dallas, TX 75219 (US)
(72) Inventor: OTTOBONI, Andrea, 45020 Giaccano con Baruchella (RO) (IT); DONNICI, Mario, 37012 Bussolengo (VR) (IT); MISTRETTA, Paolo, 37121 Verona (IT); ROSS, John David, Ovilla, TX 75154-5832 (US); STANDEFER, Karen Divita, Flower Mound, TX 75022 (US); SAMCHUKOV, Mikhail, Coppell, TX 75019 (US); CHERKASHIN, Alexander, Flower Mound, TX 75022 (US)
(74) Representative: Botti & Ferrari S.p.A.

(56) References cited:
- WO-A1-2009/105479
- WO-A1-2022/024133
- WO-A1-2022/254320
- US-A1- 2002 010 465
- US-A1- 2007 225 704

## Description

### Technical field

The present disclosure relates to the general field of external fixation, and more specifically, to the connection between external fixator struts and dedicated tools employed for the incremental or decremental adjustment of such struts, during preoperative, intraoperative and postoperative periods.

### Background

Without limiting the scope of the present disclosure, its background is herein described in connection with external fixation devices and related tools for the adjustment of struts or other connection rods thereof.

Generally speaking, external fixation devices are commonly used in a variety of surgical procedures including limb fracture fixation, lengthening and deformity correction. The process involves the application of a rigid framework. This can be a circular system, such as a hexapod system and/or a circular system with a plurality of struts. Such systems usually comprise several rings or arches that are placed externally around the limb and attached to bone segments using wires and half pins inserted into the bone segments and connected to the related section of the external rigid framework. Also, monolateral systems, such as monolateral rails or monolateral unibody, can be employed.

Rings of the rigid framework located opposite to one another are interconnected by either threaded and/or telescopic struts directly or in conjunction with uniplanar or multiplanar hinges, which allows to adjust position of the rings relative to each other longitudinally, rotationally, horizontally or angularly over a period of time.

For example, in limb lengthening, the bone is surgically divided into two segments and wires and half pins are inserted into bone segments above and below the surgical bone cut and attached to rings of a rigid framework interconnected by struts or telescopic connection struts.

For limb lengthening, the opposite rings are preferably interconnected directly by at least three or four threaded or telescopic struts that are regularly adjusted in length and allowed for gradual separation of bone segments longitudinally.

The rigid framework is used to gradually push the two bone segments apart longitudinally over a period of time (for instance, one millimeter a day). This allows the new bone to gradually form in the gap between bone segments created by this distraction technique. Once the desired amount of lengthening is achieved (e.g., 5-6 cm), the external apparatus is stabilized into a fixed position and left on the bone segments until complete mineralization of the newly formed bone (e.g., 3-6 months, depending on the nature of pathology and/or amount of lengthening).

Similarly, in deformity correction, the bone is surgically divided (usually at the apex of the deformity) into two segments and wires and half pins are inserted into bone segments above and below the surgical bone cut and attached to rings of a rigid framework. In this case also opposite rings of the rigid framework are connected together by threaded struts with attached hinges and angular distractor that is used to gradually push the two bone segments apart angularly over a period of time.

One common fixation device is a circular metal structure known as the Ilizarov apparatus. The Ilizarov apparatus, when used for limb lengthening or deformity correction, consists of several rings or arches that are placed externally around the limb and attached to surgically separated bone segments using wires and half pins. For angular deformity correction, the opposite rings of the Ilizarov apparatus are connected by a pair of hinges that provide an axis of rotation for bone segments and an angular distractor that gradually pushes two rings and associated bone segments apart.

Another common external fixation device is known as Taylor Spatial Frame, which is a hexapod type external fixation device based on a so-called Stewart platform but shares many components and features of the Ilizarov apparatus.

The Taylor Spatial Frame comprises two external fixation rings attached to bone segments by wires and half pins and connected together by five or six telescopic struts with multi-planar hinges located at both ends of the struts. Each strut may be lengthened or shortened as necessary to either pull two interconnected ring segments towards each other or push them apart.

Other examples of fixation devices of this kind are commercially known as TrueLok and Sheffield.

Adjustment of strut length allows manipulating with bone segments acutely or gradually in several axes to perform limb lengthening and correct angular, translational and rotational deformities simultaneously.

The amount of daily strut length adjustment is usually calculated by a dedicated software. Once the apparatus is attached to the bone segments, numerous parameters such as deformity parameters, frame parameters, and mounting parameters and so forth are entered into the software to characterize one ring position relative to another ring and position of bone segments relative to each other and to the rings. After calculation of the total amount of each strut length adjustment, the software provides a tabled instruction ("prescription") on the amount of each strut length adjustment that should be achieved per each increment, including a number identifying the single strut, the amount of adjustment required, and the time scheduled for such and adjustment. In most cases of deformity correction, the struts are adjusted in different directions (shortening/lengthening) and in different amounts.

Since the prescription requires several adjustments over time, usually up to four adjustments per day, most of the time these regulations cannot be performed by the surgeon or by a dedicated practitioner. The task of following the prescription is thus entrusted to the patient or to one of their relatives, which do so by turning an adjustment knob on the struts or by turning the nuts of the threaded rods with a wrench.

This way of strut length adjustment is time consuming (e.g., due to loosening and retightening of the threaded rod nuts before and after each adjustment), does not provide precise length adjustment (e.g., due to difficulty to monitor small amounts of adjustments) and creates overall frame instability during adjustments (e.g., due to dimensional clearance between connection elements).

Furthermore, the prescription for length adjustments can be complicated, and human errors are prone to occur during the course of a complicated prescription. Even though the patient is invited to verify compliance between prescription and frame status by inspecting the strut length, an error can well remain unnoticed, due to a negligent check or a total lack thereof.

Additionally, it must be considered that the feedback to the surgeon depends on the patient, who is demanded to communicate the adjustments made on the struts, usually by uploading the information on a dedicated portal. Once again, carelessness by the patient may result in incorrect or incomplete information given to the surgeon.

It is easy to understand that errors in carrying out the prescription, particularly if they are not promptly identified by the surgeon, may result in detrimental effects on the final outcome of the correction process.

To alleviate the aforementioned drawbacks, in recent year a programmable tool has been proposed for incrementally adjusting the length of the external fixator strut. The tool, described for instance in prior art application WO 2009/105479 in the name of Texas Scottish Rite Hospital for Children, is designed as an electric wrench meant to engage with the adjustment mechanism of the external fixator struts. The tool has an internal memory for storing the adjustment parameters of the prescription and is set to automatically adjust each of the struts according to said parameters.

Even though it provides numerous advantages over the previously discussed prior art, the programmable tool has however remaining drawbacks, in particularly relating to the ease of use of the device.

Indeed, in order to correctly adjust the length parameters of the struts according to the memorized prescription, it is advisable to feed the tool with real-time measurements of the struts' length. If the tool were to implement the length increments or decrements according to the prescription without feedback, the adjustment process could easily get flawed due to incremental errors, and any erroneous action by the patient - for instance coupling the tool to the wrong strut when applying the prescription - would probably disrupt the correct application of the adjustment plan over time. Therefore, the programmable tool is preferably provided with means to measure the strut, for instance a digital ruler which is meant to be coupled to the opposite ends thereof. However, such measurement means add to the complexity of the device, and more importantly they require a positive action by the patient, thus making the process of applying the prescription more difficult and prone to human error.

It is observed that alternative methods of measuring the strut, for instance through a sensor housed on-board of the strut itself, have been ruled out to date due to the difficulty in powering the internal sensor and communicating the sensor signal to the tool. In particular, wireless methods are difficult to implement due to practical design concerns and regulatory compliance, while the addition of a wired plug appears to be impractical both in terms of product design and usability.

Also, the programmable tool preferably requires further interface means, such as means to identify the struts, to help the patient in correctly performing the prescription. This means can be for instance an RFID reader meant to read a unique identification code from the strut. However, this leaves a degree of uncertainty in the process since the patient might erroneously omit the step of identifying the strut or else correctly identifying the strut and then erroneously apply the prescribed increment or decrement in length to a neighboring strut.

The technical problem underlining the present invention is that of providing a new system for adjusting external fixation that solves, or at least alleviates, the drawbacks identified with respect to the prior art.

A main aim of the improved system of the present disclosure is that of enhancing the automation of the adjustment process, without burdening to the user with additional tasks such as identification of the strut number and external measurement of the strut length, without resorting to wireless data communication between the adjustment tool and the strut.

### Summary of the Invention

The solution idea at the basis of the present disclosure is that of integrating at least a data port within the mechanical interface between the programmable tool and the strut. The data port can thus be employed to communicate the readings of an internal sensor to the controller that drives the programmable tool.

According to such a solution idea, the technical problem underlying the invention is solved by a programmable tool operable to adjust an external fixation strut having a rotatable adjustment mechanism for length adjustment, the programmable tool comprising:
an output shaft;
a motor operable to rotate said output shaft;
a first coupling portion solidly attached to said output shaft and adapted to releasably engage with a corresponding second coupling portion of the rotatable adjustment mechanism, thus enabling torque transmission from the motor to the rotatable adjustment mechanism;
a controller configured for driving said motor according to a prescription comprising instructions for adjusting the external fixation strut;
a first connecting portion in electrical communication with said controller and adapted to electrically connect with a second connecting portion of the rotatable adjustment mechanism when the first coupling portion is engaged with the second coupling portion, thus enabling data transmission from the external fixation strut to the controller.

The data transmission may be conveniently exploited by housing one or more sensors - in particular: a position sensor for length measurement - in the strut.

Therefore, the controller is configured to retrieve through the first connecting portion at least a measurement indicative of a length of the external fixation strut.

The controller can be further configured to employ said measurement indicative of a length of the external fixation strut for feedback control in driving the motor according to the prescription.

Further, the controller can use the measurement to update a status of the strut which can be sent to the surgeon making it possible to monitor the application of the prescription.

Further, the strut can be equipped with a read-only memory, containing for instance a unique code for the identification of the strut throughout the length adjustment operation. By retrieving the identification code through the provided data transmission, the controller of the programmable tool can automatically check whether the tool is coupled to the right strut and inhibit operation in the negative.

In an embodiment, the controller can even retrieve and apply the correct increment/decrement from the prescription in view of the strut which has been identified.

Preferably, the programmable tool further comprises a power supply and the electrical connection between the first connecting portion and the second connecting portion further enables power transmission to the external fixation strut.

Therefore, the electrical interface has the dual function of providing both sensor powering and data communication.

The programmable tool is a wrench.

Therefore, the first coupling portion and the second coupling portion are a wrench socket and a tip matching the wrench socket, or the other way around. By way of example, the wrench socket can be a hex-socket, while the tip can be a hex-key.

Preferably, outer engagement surfaces are provided externally with respect to the first and second coupling portions, to transmit a resistant counter torque while the driving torque is transmitted from the wrench to the rotatable adjustment mechanism.

The first connecting portion is peripheral with respect to the wrench socket or tip making up the first coupling portion. In an embodiment, it can be construed as the outer engagement surface of the first coupling portion.

The programmable tool further comprises a front muzzle featuring an annular recess surrounding the wrench socket or tip making up the first coupling portion, said first connecting portion comprising at least one inner connector, placed at a bottom of the annular recess, and at least one outer connector, placed on an outer lateral surface of said annular recess.

In an enhanced variant of the invention, at least one inner connector is a pogo pin projecting from the bottom of the annular recess and the at least one outer connector is a clip or leaf spring connector projecting from the outer lateral surface of the annular recess.

Preferably, the pogo-pins and the spring leaf connectors are a plurality, equally angularly spaced along a circumference.

In another variant of the invention, the inner and outer connectors may be simply defined by annular or cylindrical contact surface, or by connectors of different type.

Preferably, the programmable tool according to the invention comprises a locking mechanism operable to secure and selectively release the engagement of the first coupling portion with the second coupling portion.

The locking mechanism makes a stable coupling of a tool's muzzle over the strut's rotatable adjusting mechanism, thus ensuring the stability of both the mechanical and electrical connection and providing a resistant counter torque for the action of the wrench tip.

In a preferred embodiment, said locking mechanism comprises a springloaded muzzle which is retractable to release peripheral latching elements engageable within an external groove of the rotatable adjustment mechanism.

The aforementioned technical problem is also solved by an external fixation strut comprising:
an elongated body comprising at least a first shaft and a second shaft moveable with respect to each other to modify the length of said elongated body;
a rotatable adjustment mechanism for moving the second shaft with respect to the first shaft thus modifying the length of the elongated body;
a second coupling portion of the rotatable adjustment mechanism adapted to releasably engage with a first coupling portion of a programmable tool to enable torque transmission.
at least a sensor adapted to perform at least a measurement which is indicative of the length of the elongated body;
a second connecting portion of the rotatable adjustment mechanism which is in electrical communication with a first connecting portion of the programmable tool when the first coupling portion is engaged with the second coupling portion, and which enables transmission of data from the sensor to the programmable tool.

A person skilled in the art will understand that the external fixator strut according to the present invention is meant to be used in combination with the programmable tool according to the present invention, since the two define a plug and socket connection wherein the latter device connects with the former.

The external fixator strut may advantageously be comprised in an external fixator assembly comprising a plurality of such struts along with at least two ring connectors interconnected by said struts, wherein adjusting the length of said fixator strut modifies the relative orientation between the ring connectors. The ring connectors are meant to be attached to bone sites via bone screws ore other fixators known in the art.

In a preferred embodiment, the rotatable adjustment mechanism of the fixation strut has a worm gear mechanism comprising a worm screw meshing with a worm gear, a worm screw shaft being integral with the second coupling portion.

Preferably, the worm gear is rotatably fixed to the first shaft and threadingly engaged with the second shaft, so that turning the worm gear about its axis cause the first shaft to translate with respect to the second shaft, eventually increasing or decreasing the length of the elongated body.

In a preferred embodiment, the second connecting portion is coaxially arranged around the second coupling portion of the rotatable adjustment mechanism.

Preferably, the rotatable adjustment mechanism comprises a protruding head housing the second coupling portion and defining the second connecting portion.

Preferably, the protruding head comprises an external groove arranged for releasable fixation by a locking mechanism of the programmable tool.

The aforementioned technical problem is also solved by a kit comprising both the programmable tool and the external fixation strut previously discussed.

### Brief description of the drawings

For a more complete understanding of the features and advantages of the present disclosure, reference is now made to the detailed description of four different struts embodiments of the invention along with the accompanying figures and in which:
FIG. 1 is a side view of a first embodiment of an external fixation strut according to the present disclosure;
FIG. 2 is a cross-sectional view of an external fixation strut, taken along the cutting plane referenced with A-A in FIG. 1;
FIG. 3 is a cross-sectional view of an external fixation strut, taken along the cutting plane referenced with B-B in FIG. 1;
FIG. 4 is a perspective view of a second embodiment of an external fixation strut according to the present disclosure;
FIG. 5 is a detail of the perspective view of FIG. 4;
FIG. 6 is a cross-sectional view of the external fixation strut of FIG.4, taken along a mid-section of the extractable tube;
FIG. 7 is a side view of a third embodiment of an external fixation strut according to the present disclosure;
FIG. 8 is a cross-sectional view of an external fixation strut, taken along the cutting plane referenced with C-C in FIG. 7;
FIG. 9 is a perspective view of an embodiment of an external fixation strut according to the present disclosure;
FIG. 10 is a side view of a fourth embodiment of an external fixation strut according to the present disclosure;
FIG. 11 is a cross-sectional view of an external fixation strut, taken along the cutting plane referenced with D-D in FIG. 10;
FIG. 12 is a cross-sectional view of an external fixation strut, taken along the cutting plane referenced with E-E in FIG. 10;
FIG. 13 is a side view of the fourth embodiment of an external fixation strut according to the present disclosure;
FIG. 14 is a cross-sectional view of an external fixation strut, taken along the cutting plane referenced with F-F in FIG. 10;
FIG. 15 is a perspective view of a fourth embodiment of an external fixation strut according to the present disclosure;
FIG. 16 is an enlarged detail of the area referenced with G in FIG. 14;
FIG. 17 is an enlarged detail of the area referenced with H in FIG. 14;
FIG. 18 is a top view of an embodiment of a programmable tool according to the present disclosure;
FIG 19 is a side view of an embodiment of an external fixation strut next to an embodiment of a programmable tool, according to the present disclosure;
FIG. 20 is a cross-sectional view of an external fixation strut next to a programmable tool, taken along the cutting plane referenced with I-I in FIG. 19;
FIG 21 is a side view of an embodiment of an external fixation strut next to an embodiment of a programmable tool, according to the present disclosure;
FIG. 22 is a cross-sectional view of an external fixation strut next to a programmable tool, taken along the cutting plane referenced with J-J in FIG. 21;
FIG. 23 is an enlarged detail of the area referenced with K in FIG. 22;
FIG. 24 is a top part of a flow chart showing operation of a programmable tool according to the present disclosure;
FIG 25 is the bottom part of the flow chart of FIG. 24.

### Detailed description

While the making and using of various embodiments of the present disclosure are discussed in detail below, it should be appreciated that the present disclosure provides many applicable inventive concepts that can be embodied in a wide variety of specific contexts. The specific embodiments discussed herein are merely illustrative of specific ways to make and use the disclosure and do not delimit the scope of the disclosure.

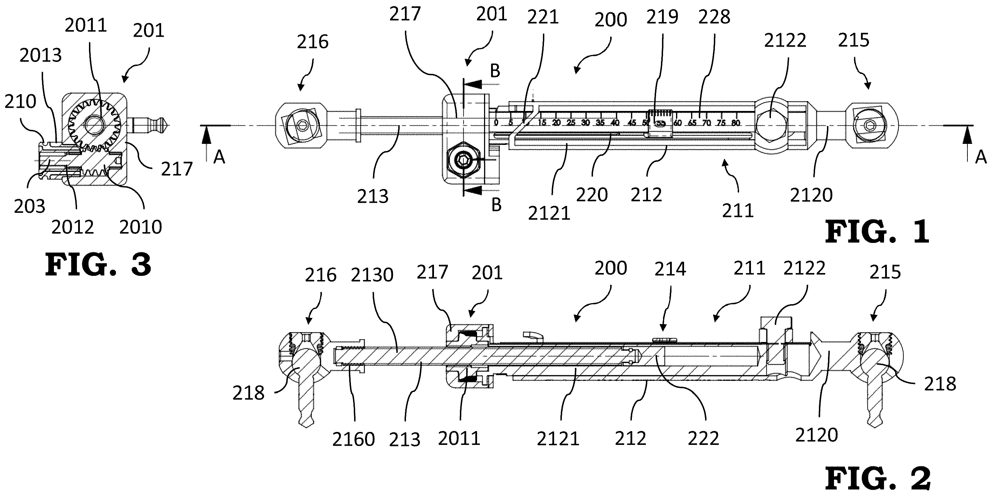

**Figure 1** shows a schematic side view of an improved external fixation strut 200 according to the present disclosure having an elongated body 211 with opposite ends 215, 216 configured to be attached to a respective fixation ring or arch of an external fixator, preferably of the hexapod type.

The rings of the body of the external fixator, which can be fixed to bone sites via half pins or wires, are known per se and not depicted in the enclosed figures.

The elongated body 211 is formed by a first hollow tubular shaft 212 in which a second tubular shaft 213 is slidably hosted. The first shaft 212 is in turn composed of a hollow main body 2120, which culminates with the first end 215, and a sliding body 2121 which slides within the main body 2120 and houses the second tubular shaft 213.

The relationship between the two bodies of the first 212 shaft and between the first shaft and the second shaft 213 confers a telescopic configuration to the ensemble of the strut 200, so that the elongated body 201 may be adjusted in its length according to the needs of keeping the interconnected rings in a predetermined relative spatial relationship.

In particular, the relationship between the main body 2120 and the sliding body 2121, which defines the length of the first shaft 212, is varied in a first regulation of the length between the two attachment points of the strut 200, while the relationship between the first shaft 212 and the second shaft 213 is varied to finely adjust the strut length, in particular in a postoperative follow-up.

The main body 2120 and the sliding body 2121 are locked together by a locking screw 2122, which is released for manual adjustment of the relative position of the two elements.

On the contrary, the position of the second shaft 213 with respect to the first shaft 212 is adjusted through a rotatable adjustment mechanism 201 contained in a casing 217 of the sliding body 2121 of the first shaft 212. The rotatable adjustment mechanism will be described in greater detail with reference to Figure 3.

An external metering cursor 219, which is solidly attached to an inner end of the second shaft 213 through a leg traversing a longitudinal slit 220 of the sliding body 2121, slides along a graduated scale 228 integral with the casing 217 and makes it possible to visually assess the relative position of the second shaft 213 with respect to the first 212.

To make the graduated scale 228 visible to the user, the frame of the hollow main body 2120 has an open side. A transverse bridge 221 of the frame is provided at an end of said open side. The bridge 221 is visible against the background of the graduated scale 228, making it possible to visually assess the relative position of the sliding body 2121 with respect to the main body 2120, i.e. the length of the first shaft 212.

**Figure 2** shows a cross-sectional view of the device of figure 1.

At the two ends 215, 216 of the strut 200, ball-joints 218 are provided which makes it possible to articulate the strut 200 with respect to a fixation ring or arch to which it is attached.

The rotation imparted through the rotatable adjustment mechanism 201 defines, through a threaded connection, a displacement of the second shaft 213 with respect to the first shaft 212, so that the second end 216 is pulled away or dragged towards the first end 215.

In particular, the second shaft 213 has an intermediate shank 2130 which is externally threaded. The intermediate shank 2130 is rotatably inserted within a sleeve 2160 of the respective end 216 of the strut 200 on one side, and within a slidable end-piece 222 on the other side.

**Figure 3** shows a cross-sectional view of the above-mentioned rotatable adjustment mechanism 201.

The rotatable adjustment mechanism 201 comprises a worm gear mechanism having a worm screw 2010 meshing with a worm gear 2011, the latter being coaxial with the second shaft 213 and threadingly engaged with it. Therefore, a rotation of the worm screw 2010 determines a corresponding rotation of the worm gear 2011 which, in turn, drives the second shaft 213 into translation with respect to the first shaft 212.

The worm screw 2010 has a worm screw shaft 2012 which is orthogonal to the axis of the elongated body 211.

A protruding head 2013, which protrudes from a lateral wall of the casing 217, rotatingly houses said screw shaft 2010 and, as will be further discussed in the following, defines a second coupling portion 203 for the attachment of a corresponding coupling portion of a wrench tool.

The protruding head 2013 further defines an outer sleeve surrounding the second coupling portion 203. In the depicted embodiment, the outer sleeve has an inner circular profile and an outer hexagonal profile, the latter comprising an external groove 210 for attachment of a locking mechanism of an adjustment tool.

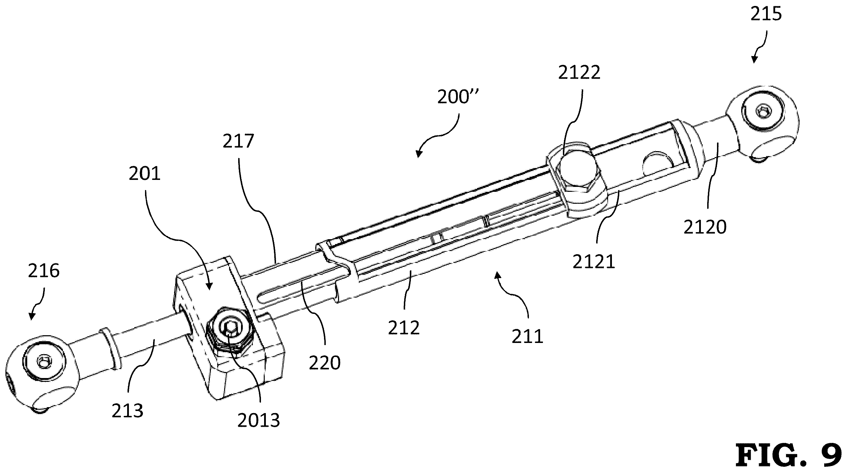

Further details of the of the attachment will be discussed with reference to the embodiment which is depicted in Figure 9 and which features an identical attachment portion.

A position sensor 214, which is visible in previously discussed Figure 2, is provided within the first shaft 212 to sense the position of the metering cursor 219. In this embodiment of the strut, the position sensor is based on inductive technology. The position sensor is glued on the external surface. The sensor is also positioned within the tubular element.

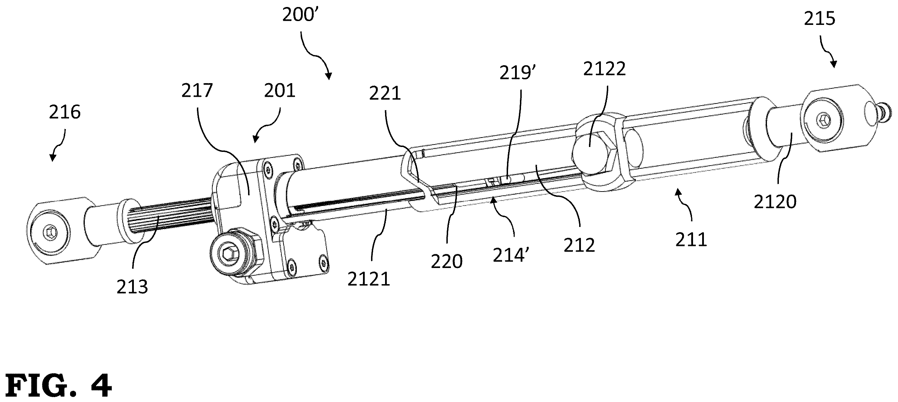

**Figure 4** refers to an alternative embodiment of an external fixation strut 200', which features an improved version of the inductive position sensor 214'.

In such an alternative embodiment, the external fixation strut 200' essentially has the same components and main features of the previous embodiments, which is previously described. Such components and features are therefore indicated in the figures by reference numerals previously used, and they are not described anew in the following paragraphs.

The inductive position sensor 214' differs from the previously described position sensor 214 mainly in that the metering cursor 219', the position of which is detected, is kept within the body of the first shaft 212: in particular, within the tubular element defined by the sliding body 2121.

The internal metering cursor 219' is preferable since it does not interfere with the readings on the external scales, and it greatly reduces the risk of damages from handling.

On the other hand, the surrounding sliding body 2121 heavily interferes with the reading of the position of the internal metering cursor 219', and the position sensor 214' needs to be accurately tuned in order to cancel out the noise.

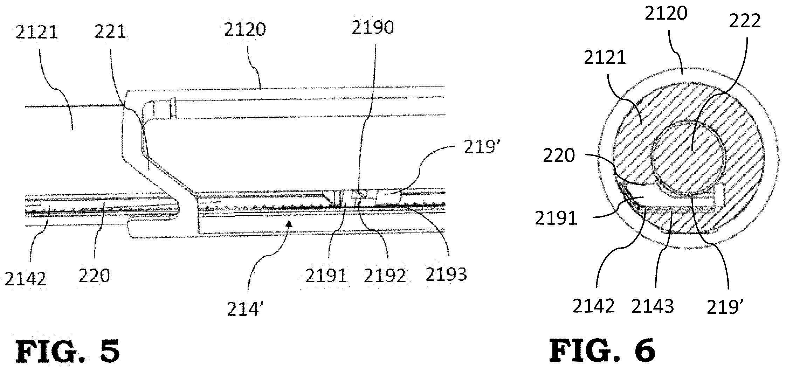

**Figure 5** shows an enlarged detail of the inductive position sensor 214' from previous Figure 4. The internal metering cursor 219' has a sled-shaped body, featuring an upper transverse groove 2190 for insertion of a corresponding tooth provided on the bottom of the otherwise cylindrical slidable end-piece 222, which is visible in Figure 6. The groove and tooth engage to define a connection so that the metering cursor 219' moves with the end-piece 222. To ensure a more stable connection, the two elements can also be glued or soldered.

In an alternative embodiment not visible in the attached Figures, the groove may not be a pass-through groove, being only present on the inner wall. Therefore, the groove is not visible from the outside. A hole can be added on the sliding body 2121 to allow gluing/welding of the cursor. The internal metering cursor 219' further features a lateral protrusion 2191 which extends at least partly through the slit 220 and which exhibits an index 2192 for visual reading of the element's position of on an underlying metered scale.

The internal metering cursor 219' comprises a copper layer 2193, gold-plated to avoid corrosion issues, which is fixed on the underside of the sled-shaped body. The copper layer 2193 is meant to amplify the antenna gain, thereby increasing the sensitivity of the metering device.

The inductive position sensor 214' further comprises a sensing strip 2142, which is obtained as a flexible printed circuit board in a *per se* known manner.

**Figure 6** shows a cross-section of the external fixation strut 200' which shows further details of the inductive position sensor 214' and of its sensing strip 2142.

The sensing strip 2142 is attached over an elongated support plate 2143 made of ferromagnetic material, for instance mu-metal. The ferrite plate 2143 is meant to shield the sensor from external interferences. The top face of the sensing strip 2142 faces the internal metering cursor 219' and the ferrite plate 2143 backs the sensing strip 2142 on the other side.

In device manufacturing, the sensing strip 2142 can be glued to the ferrite plate 2143 via a double-sided adhesive strip which is provided on the sensing strip 2142. Then, the combined plate-and-strip are inserted within the sliding body 2121 through the slit 220. As an alternative, the ferromagnetic plate is directly provided as a last layer of the sensing strip.

The position sensor 214' is preferably an absolute sensor.

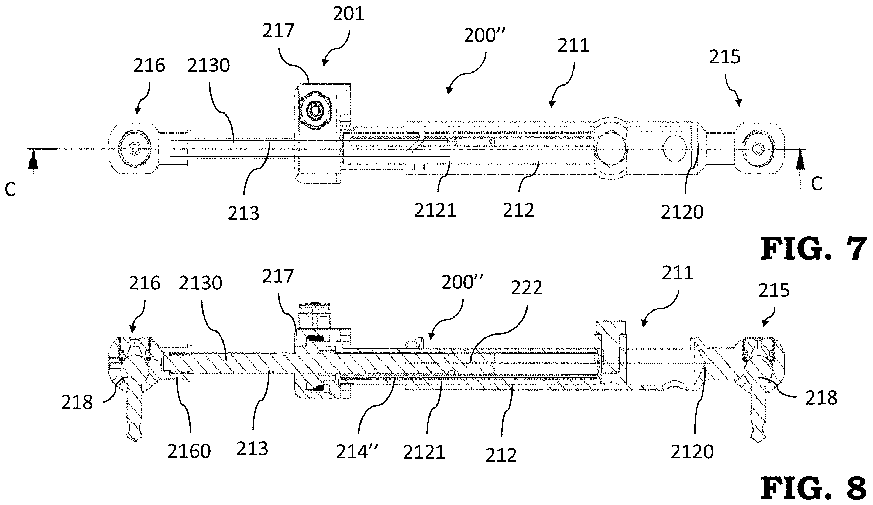

**Figure 7** shows a schematic side view of a third embodiment of the external fixation strut 200" according to the invention.

The third embodiment is identical to the previous ones except for the position sensor 214", which is based on a capacitive technology.

**Figure 8** shows a cross-sectional view of the device of figure 7.

The position sensor 214" is glued on an internal surface of the sliding body 2121, and it measures the relative position of the end-piece 222.

The position sensor 214" is therefore housed within the casing 217, solidly attached to it and it extends along the longitudinal inner length of the first shaft 212.

The position sensor 214" detects an absolute position of the end-piece 222 of the second shaft 213, returning a measurement value which can be employed to assess the relative position of the second shaft 213 with respect to the first shaft 212.

**Figure 9** shows a schematic perspective view of the external fixation strut 200" according to the third embodiment of the invention, with no further elements disclosed.

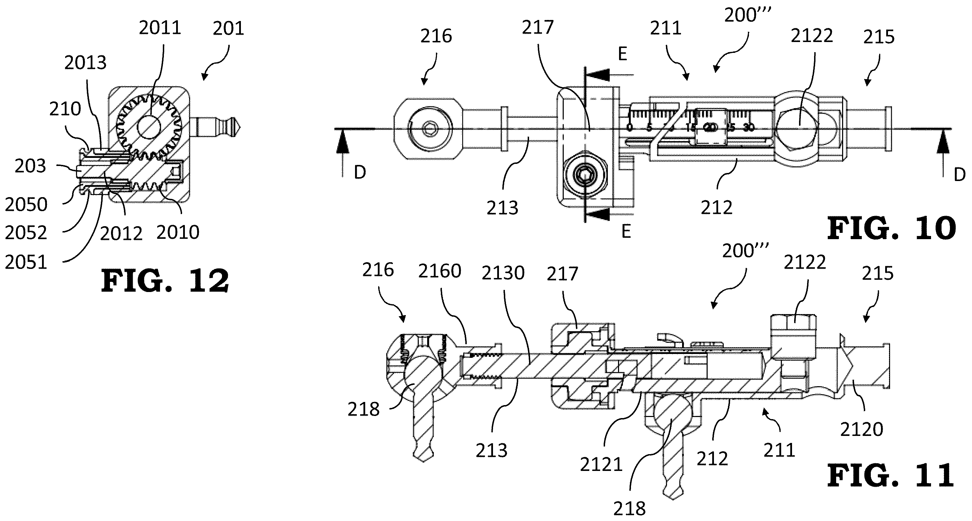

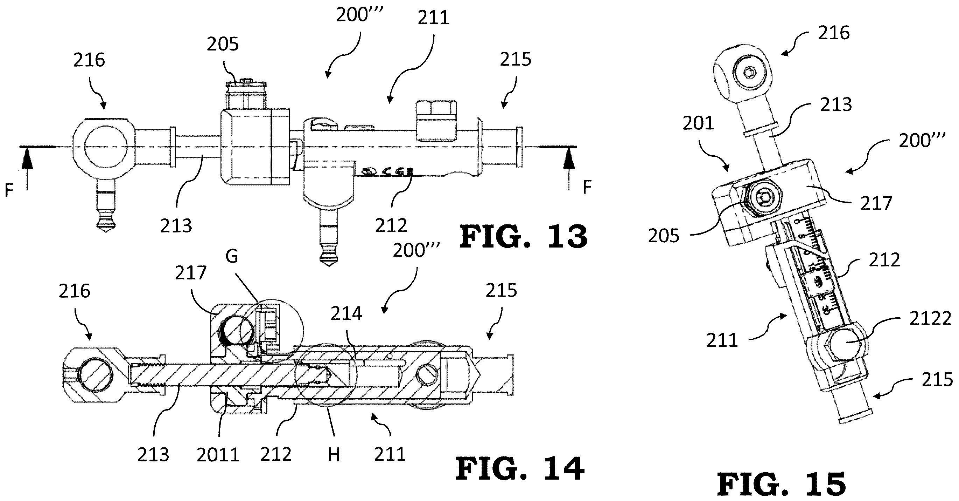

**Figures 10-15** refer to fourth embodiment of an external fixation strut 200"', which is shorter in size with respect to the long external fixation strut 200' according to the first embodiment previously discussed.

In such an alternative embodiment, the external fixation strut 200‴ essentially has the same components and main features of the longer embodiments, which are previously described. Such components and features are therefore indicated in the figures by reference numerals previously used, and they are not described anew in the following paragraphs.

The main structural difference with respect to the longer version is that here the two attachment points defined by the ball-joints 218 are set closer to each other, thanks to the fact that the ball-joint of the first shaft 212 is not placed at the end 215 thereof, but rather at an opposite end of the hollow main body 2120 of the first shaft 215.

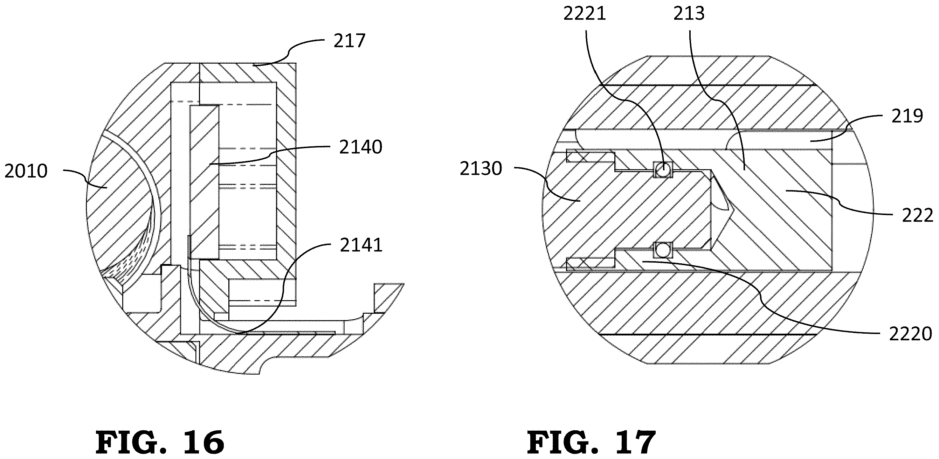

**Figure 16** shows a detail in the construction of the previously introduced position sensor 214.

A flexible signal line 2141 of the position sensor, made in form of a flat cable, connects an electronic board 2140 to the body of the sensor 214, which extends in the vicinity of the sensed end-piece 222.

The electronic board 2140 is meant for sensor powering and data transmission and is meant to connect to a programmable tool 100 via the connecting system which is described in the following.

It is observed that the flexible signal line 2141 has at least one curvature to connect the rotatable adjustment mechanism's housing to the rest of the shaft. Said curvature is designed such that the radius is kept above a threshold value to avoid damaging the signal line during the manufacturing process.

The same construction of the electronic board 2140 with flexible signal line 2141 is also preferably adopted in the position sensors 214', 214" of the second and third embodiments.

**Figure 17** shows a detail in the construction of the previously introduced end-piece 222.

As seen in the Figure, the end of the shank 2130 is inserted in a sleeve 2220 of the end-piece 222 in a rotatable manner, through an interposed ring 2221. The ring 2221 allows the end-piece 222 and thus the cursor 219 to translate along the longitudinal axis of the strut 200"' while the shank 2130 rotates.

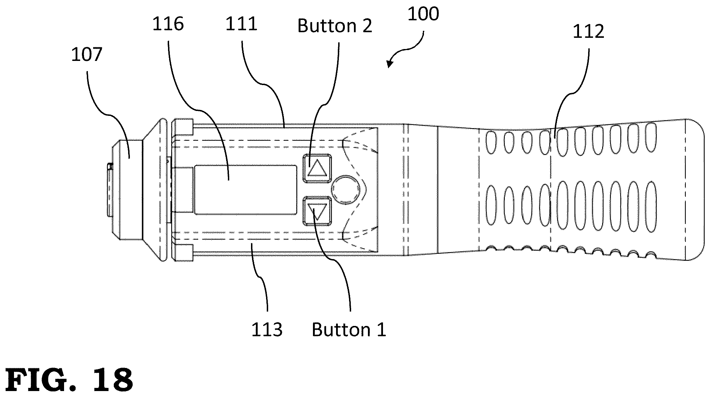

**Figure 18** shows a top view of a programmable tool 100 according to the present disclosure, which is in the form of an electric wrench.

The wrench comprises a rigid external casing 111.

In the depicted embodiment, the casing 111 has a substantially cylindrical shape. However, the shape of the housing 111 may be any shape and size convenient for use.

In the depicted embodiment, the casing 111 defines a handpiece having a distal gripping portion 112 with an ergonomic handle followed by a proximal interface portion 113.

The housing may have a variety of connection ports, connectors, display and controls.

In the depicted embodiment, the interface portion comprises a first and a second pushbutton, identified as Button 1 and Button 2, and a display 116, and further has an annular LED indicator which can be lit in various colors and in a steady or pulsing mode in order to signal a plurality of device statuses to the user.

In a preferred embodiment, the annular LED indicator may have up to four lighted states, respectively signaling: a call to action by the user, an ongoing operation by the wrench, successful or unsuccessful completion of a wrench operation.

The LED indicator is preferably covered by a transparent light guide which is integrated into the casing 111.

The device may further comprise a buzzer or any other audio device.

The device further comprises a front muzzle 107 ahead of the interface portion 113, which is arranged to connect to the torque input port of the rotatable adjustment mechanism 201 of the external fixation struts 200, 200'.

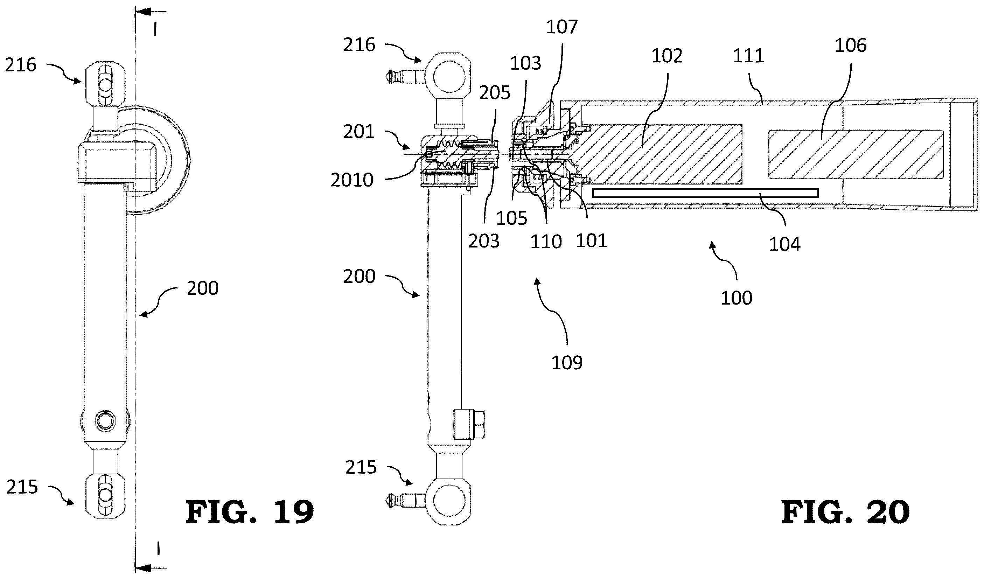

**Figure 19** shows a side view of the programmable tool 100 about to be coupled with an external fixation strut 200.

**Figure 20** is a cross-sectional view of the two devices of figure 19, and schematically shows the main components inside the casing 111 of the programmable tool 100.

The programmable tool 100 comprises power source 106 in the form of a rechargeable battery, an electric motor 102 which preferably comprises a gearbox in-line with a coaxial output shaft 101, and a controller 104 in the form of a PCB.

The battery 106 is preferably recharged wirelessly, through inductive technology, to minimize electrical hazards for the user.

The programmable tool 100 may also comprise an internal memory, which can be conveniently used for storing a patient's prescription and an actual length of a plurality of struts making up an external fixator to be adjusted by the tool 100.

The programmable tool 100 has means of communicating with an external data source, in the form of data ports and/or wireless connectivity. Preferably, the programmable tool as a SIM housing (not shown) which is meant to provide internet connectivity to the device.

The controller 104 of the programmable tool 100 is adapted to communicate with an external portal, directly or via a portable electronic device such as a smartphone, to retrieve a surgeon's prescription and update a status comprising at least the length of the several external fixation struts 200, 200', 200", 200‴ of an external fixator.

The controller 104 is connected to the pushbuttons 114, 115, display 116 and annular LED indicator in order to read user's commands, communicate status updates, or guide the user throughout a process of adjusting all of the struts 200, 200', 200", 200‴ according to a given prescription.

The muzzle 107 of the programmable tool 100 houses the output shaft 101, which culminates with a first coupling portion 103 in the form of a wrench socket, in particular a hex socket.

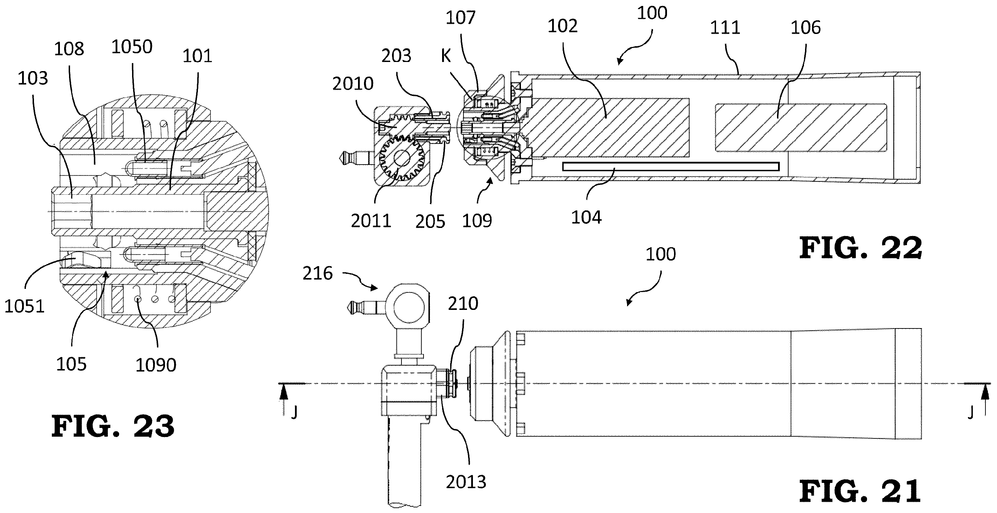

The muzzle further comprises a locking mechanism 109 operable to secure and selectively release the engagement of the first coupling portion 103 with the second coupling portion 203. When the locking mechanism 109 is engaged the protruding head 2013 of the rotatable adjustment mechanism 201 is housed within an annular recess 108 of the muzzle 107, best shown in Figure 23.

The locking mechanism 109 is defined by the body of the muzzle 107, which may be retracted towards the programmable tool 100 housing 111 by pressing it against elastic means, which may be in the form of a spring 1090 best shown in Figure 23. The muzzle 107 internally has latching elements 110, which may be in the form of rollers moving along oblique paths, said roller being externally biased by the action of the spring 1090 in order to engage the first coupling portion 103.

Around said first coupling portion 103 is provided a first connecting portion 105, which is meant to electrically connect with a second connecting portion 205 of the strut 200, 200'.

When the locking mechanism 109 is engaged, the first connecting portion 105 is connected to the second connecting portion 205 of the strut 200, 200' and data connection is provided which allows the controller 104 to retrieve data from sensor 214.

The electric connection further ensures powering of the sensor 214 from the power source 106.

**Figure 21** shows a further side view of the programmable tool 100 about to be coupled with an external fixation strut 200.

**Figure 22** is a cross-sectional view of the two devices of figure 18.

**Figure 23** is an enlargement of figure 19, which shows a detail of the first connecting portion 105.

The first connecting portion 105 comprises a plurality of inner connector 1050 at the bottom of the annular recess 108, which are preferably in the form of pogo-pins, meant to connect a first electric pole.

Further, the first connecting portion 105 comprises a plurality of outer connectors 1051, preferably in the form of clip, tape spring or leaf spring connectors, projecting from the outer lateral surface of said annular recess 108. Said outer connectors 1051 are meant to connect a second electric pole.

In use, the inner connectors 1050 contact an inner surface 2050 of the protruding head 2013 outer sleeve, while the outer connectors 1051 contact an outer surface 2051 of the same sleeve. As best seen in Figure 12, said inner surface 2050 and outer surface 2051 are separated by a dielectric layer 2052.

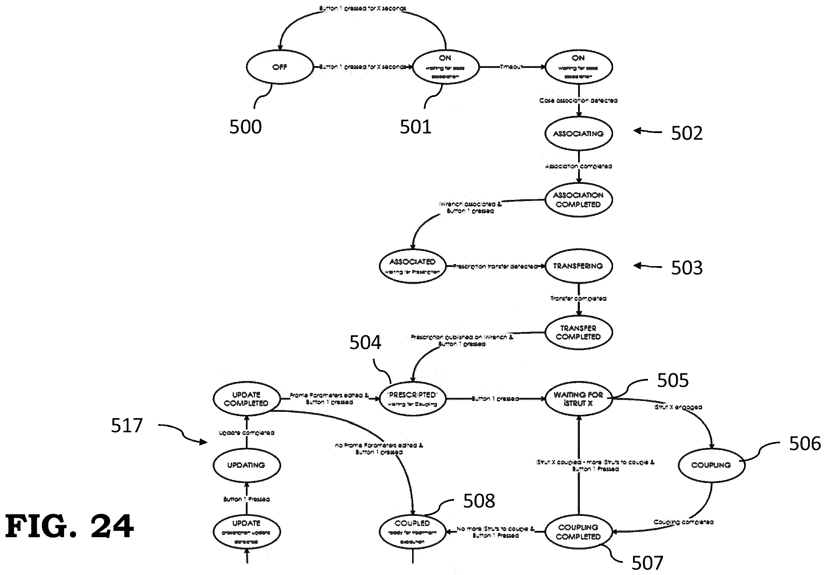

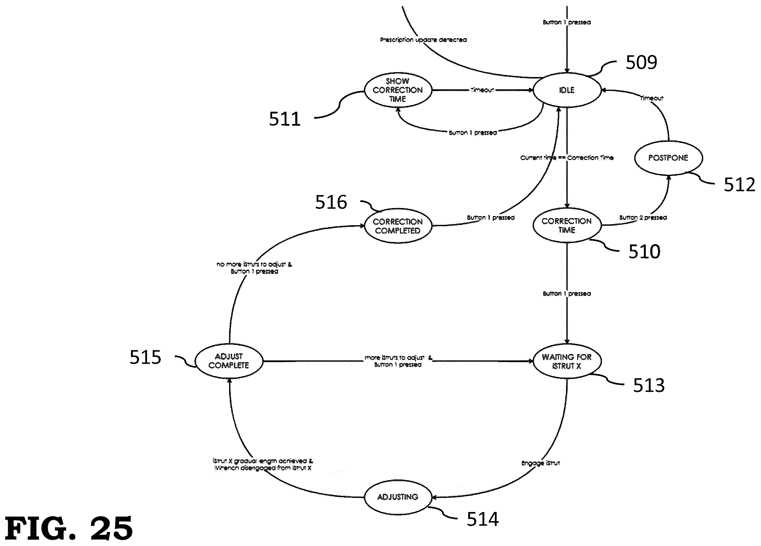

**Figure 24** **and** **25** show a top and a bottom part showing a flow diagram of an operational method of the programmable tool 100.

The device can be turned from an off state 500 to an on state 501 by the user, for instance by pressing Button 1 or Button 2 for a given time.

A similar user action may be demanded to return the device to the off state 500.

After a given time from being turned on, the device will be associated to a specific case 502.

Then, after a command by the user, the device will connect to the internet and retrieve from a dedicated portal the prescription for the patient's case 503.

After the prescription has been uploaded to the internal memory, the device is in a general waiting state 504 which may be signaled to the user via the display 116 and/or the annular LED indicator.

Upon a user command, for instance the pressure of Button 1, the device is in a coupling waiting state 505 and it will signal through the display 116 that it is ready to connect with a given strut.

In coupling waiting state 505, the user is expected to mechanically couple the device to a strut 200, 200' which, as previously discussed, also results in a data connection.

When the data connection is performed 507, the device advises the user which may then couple the other struts 200, 200' in a similar way.

Once all struts 200, 200' have been correctly coupled, the device enters a coupled waiting state 508.

Then, upon a command by the user, the device enters an idle state 509 ready to signal when a prescribed correction time 510 is reached. In such a state, the user can interrogate the device - for instance, by pressing Button 1 - which will then display the next correction time 511.

When the correction time 510 is eventually reached, the user may be advised via an audio and/or a visual signal that he/she is required to take action.

The user will then be granted the choice of either performing or postponing 512 the adjustment of the struts. Button 1 and Button 2 can be alternately employed to discriminate between the two choices.

If the user decides to perform the correction, the device will then guide the user toward engaging the struts 200, 200' to be adjusted 513. Once a strut 200, 200' is coupled, the device will automatically perform the adjustment in an adjustment step 514, and thereafter signal to the user that the adjustment is complete 515.

If another strut is to be adjusted, steps 513-515 are repeated; otherwise, the device signals to the user that the correction is completed 516, and returns to the idle state 509.

From the idle state 509, the device is also able to update the prescription 517 whenever a prescription update is detected.

It will be understood that particular embodiments described herein are shown by way of illustration and not as limitations of the disclosure. The principal features of this disclosure can be employed in various embodiments without departing from the scope of the disclosure. Those skilled in the art will recognize, or be able to ascertain using no more than routine experimentation, numerous equivalents to the specific devices and procedures described herein.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

## Claims

1. A programmable tool (100) operable to adjust an external fixation strut (200, 200', 200", 200"') having a rotatable adjustment mechanism (201) for length adjustment, the programmable tool comprising:
an output shaft (101);
a motor (102) operable to rotate said output shaft (101);
a first coupling portion (103) solidly attached to said output shaft (101) and adapted to releasably engage with a corresponding second coupling portion (203) of the rotatable adjustment mechanism (201), thus enabling torque transmission from the motor (102) to the rotatable adjustment mechanism (201); wherein the first coupling portion (103) and the second coupling portion (203) are a wrench socket and a tip matching the wrench socket, or the other way around;
a controller (104) configured for driving said motor (102) according to a prescription comprising instructions for adjusting the external fixation strut (200, 200', 200", 200‴);
a first connecting portion (105) in electrical communication with said controller (104) and adapted to electrically connect with a second connecting portion (205) of the rotatable adjustment mechanism (201) when the first coupling portion (103) is engaged with the second coupling portion (203), thus enabling data transmission from the external fixation strut (200, 200', 200", 200‴) to the controller (104); wherein said first connecting portion (105) is peripheral with respect to the wrench socket or tip making up the first coupling portion (103);
**characterized in that** it further comprises a front muzzle (107) featuring an annular recess (108) surrounding the wrench socket or tip making up the first coupling portion (103), said first connecting portion (105) comprising at least one inner connector (1050), placed at a bottom of the annular recess (108), and at least one outer connector (1051), placed on an outer lateral surface of said annular recess (108).

2. The programmable tool (100) of claim 1, wherein it further comprises a power supply and the electrical connection between the first connecting portion (105) and the second connecting portion (205) further enables power transmission to the external fixation strut (200, 200', 200", 200‴).

3. The programmable tool (100) of claim 1, wherein the at least one inner connector (1050) is a pogo pin projecting from the bottom of the annular recess (108) and the at least one outer connector (1051) is a clip or leaf spring connector projecting from the outer lateral surface of the annular recess (108).

## Patentansprüche

1. Programmierbares Werkzeug (100), das betreibbar ist, eine externe Befestigungsstrebe (200, 200', 200", 200‴) einzustellen, die einen drehbaren Einstellmechanismus (201) zur Längeneinstellung aufweist, wobei das programmierbare Werkzeug Folgendes umfasst:
eine Ausgangswelle (101);
einen Motor (102), der betreibbar ist, die Ausgangswelle (101) zu drehen;
einen ersten Kopplungsabschnitt (103), der an der Ausgangswelle (101) fest befestigt ist und ausgelegt ist, mit einem entsprechenden zweiten Kopplungsabschnitt (203) des drehbaren Einstellmechanismus (201) lösbar in Eingriff zu gelangen, wobei eine Drehmomentübertragung vom Motor (102) auf den drehbaren Einstellmechanismus (201) ermöglicht wird; wobei der erste Kopplungsabschnitt (103) und der zweite Kopplungsabschnitt (203) ein Steckschlüsseleinsatz bzw. eine zum Steckschlüsseleinsatz passende Werkzeugspitze, oder umgekehrt, sind;
eine Steuereinheit (104), die zum Antreiben des Motors (102) gemäß einer Vorschrift, die Anweisungen zum Einstellen der externen Befestigungsstrebe (200, 200', 200", 200"') umfasst, konfiguriert ist;
einen ersten Verbindungsabschnitt (105) in elektrischer Verbindung mit der Steuereinheit (104) und der ausgelegt ist, mit einem zweiten Verbindungsabschnitt (205) des drehbaren Einstellmechanismus (201) elektrisch zu verbinden, wenn sich der erste Kopplungsabschnitt (103) mit dem zweiten Kopplungsabschnitt (203) in Eingriff befindet, womit eine Datenübertragung von der externen Befestigungsstrebe (200. 200', 200", 200"') zur Steuereinheit (104) ermöglicht wird; wobei der erste Verbindungsabschnitt (105) in Bezug auf den Steckschlüsseleinsatz oder die Werkzeugspitze, der bzw. die den ersten Kopplungsabschnitt (103) bildet, außerhalb liegt;
**dadurch gekennzeichnet, dass** es ferner eine vordere Mündung (107) umfasst, die eine ringförmige Vertiefung (108) bietet, die den Steckschlüsseleinsatz oder die Werkzeugspitze, der bzw. die den ersten Kopplungsabschnitt (103) bildet, umgibt, wobei der erste Verbindungsabschnitt (105) mindestens einen Innenverbinder (1050), der am Grund der ringförmigen Vertiefung (108) angeordnet ist, und mindestens einen Außenverbinder (1051), der auf einer äußeren Seitenfläche der ringförmigen Vertiefung (108) angeordnet ist, umfasst.

2. Programmierbares Werkzeug (100) nach Anspruch 1, wobei es ferner eine Stromversorgung umfasst und die elektrische Verbindung zwischen dem ersten Verbindungsabschnitt (105) und dem zweiten Verbindungsabschnitt (205) ferner eine Energieübertragung auf die externe Befestigungsstrebe (200, 200', 200", 200‴) ermöglicht.

3. Programmierbares Werkzeug (100) nach Anspruch 1, wobei der mindestens eine Innenverbinder (1050) ein Federkontakt ist, der vom Grund der ringförmigen Vertiefung (108) vorsteht, und der mindestens eine Außenverbinder (1051) ein Klammerverbinder oder ein Blattfederverbinder ist, der von der äußeren Seitenfläche der ringförmigen Vertiefung (108) vorsteht.

## Revendications

1. Outil programmable (100) utilisable pour ajuster une tige de fixation externe (200, 200', 200", 200"') ayant un mécanisme d'ajustement rotatif (201) pour l'ajustement de la longueur, l'outil programmable comprenant:
un arbre de sortie (101);
un moteur (102) utilisable pour faire tourner ledit arbre de sortie (101);
une première portion d'accouplement (103) fixée solidement audit arbre de sortie (101) et adaptée pour s'engager de manière amovible avec une deuxième portion d'accouplement (203) correspondante du mécanisme d'ajustement rotatif (201), permettant ainsi la transmission du couple à partir du moteur (102) au mécanisme d'ajustement rotatif (201); la première portion d'accouplement (103) et la deuxième portion d'accouplement (203) étant une douille de clé et une pointe correspondant à la douille de clé, ou vice versa;
un contrôleur (104) configuré pour entraîner ledit moteur (102) selon une prescription comprenant des instructions pour ajuster la tige de fixation externe (200, 200', 200", 200"');
une première portion de connexion (105) en communication électrique avec ledit contrôleur (104) et adaptée pour se connecter électriquement à une deuxième portion de connexion (205) du mécanisme d'ajustement rotatif (201) lorsque la première portion d'accouplement (103) est en prise avec la deuxième portion d'accouplement (203), permettant ainsi la transmission de données à partir de la tige de fixation externe (200, 200', 200", 200"') au contrôleur (104); ladite première portion de connexion (105) étant périphérique par rapport à la douille de clé ou à la pointe composant la première portion d'accouplement (103);
**caractérisé en ce qu'**il comprend en outre une embouchure avant (107) comportant un évidement annulaire (108) entourant la douille de clé ou la pointe composant la première portion d'accouplement (103), ladite première portion de connexion (105) comprenant au moins un connecteur interne (1050), placé sur un fond de l'évidement annulaire (108), et au moins un connecteur externe (1051), placé sur une surface latérale externe dudit évidement annulaire (108).

2. Outil programmable (100) selon la revendication 1, comprenant en outre une alimentation électrique et la connexion électrique entre la première portion de connexion (105) et la deuxième portion de connexion (205) permet en outre la transmission de puissance à la tige de fixation externe (200, 200', 200", 200"').

3. Outil programmable (100) selon la revendication 1, dans lequel l'au moins un connecteur interne (1050) est une broche pogo faisant saillie du fond de l'évidement annulaire (108) et l'au moins un connecteur externe (1051) est un connecteur à clip ou à ressort à lame faisant saillie de la surface latérale externe de l'évidement annulaire (108).
